# EUROPEAN PATENT APPLICATION

(11) **EP 4 643 758 A1**
(43) Date of publication of application: **05.11.2025**
(21) Application number: 24745340.0
(22) Date of filing: 24.01.2024
(51) Int. Cl.: A61B 1/04, A61B 1/06, A61B 1/00

(54) **ENDOSCOPE SHEATH PROTECTION SYSTEM**

(30) Priority: 18.01.2024 CN 202410077191
(71) Applicant: Anhui Dendrite Optical Technology Co., Ltd., Hefei Cty, Anhui Province (CN)
(72) Inventor: CHIANG, Li-yang, Jingan District Shanghai 200040 (CN); WENG, Xianjie, Jingan District Shanghai 200040 (CN)
(74) Representative: Winter, Brandl - Partnerschaft mbB
(86) International application number: PCT/CN2024/073849
(87) International publication number: WO 2025/152200

(57) **Abstract**

A protection structure for a lens sheath of an endoscope is provided, including an incident light module, an objective lens module, an imaging module, and a lens sheath coaxial module. The incident light module is used to provide an incident light source, which strikes onto a surface of an object under observation after passing through the objective lens module, and then after being reflected by the surface of the object under observation, passes through the objective lens module again and propagates backward to the imaging module for imaging processing. The lens sheath coaxial module includes a base, an internal lens sheath fixedly connected to the base, and an external lens sheath sleeved on an outside of the internal lens sheath and fixedly connected to the base, the objective lens module is fixedly disposed at a front end of the internal lens sheath, and the external lens sheath comprises a protective lens fixedly disposed at a front end of the external lens sheath.

## Description

### CROSS REFERENCE TO RELATED APPLICATION

This application claims priority to Chinese Patent Application No. 202410077191.5, filed with CNIPA on January 18, 2024, entitled as "PROTECTION STRUCTURE FOR LENS SHEATH OF ENDOSCOPE", the entire content of which is incorporated herein by reference in its entirety.

### TECHNICAL FIELD

The present disclosure relates to the field of medical devices, and in particular, to a protection structure for a lens sheath of an endoscope.

### BACKGROUND

An endoscope is a device for examining and treating the internal tissues of the human body by means of a head probe and an optical lens. The optical imaging principle is that light enters the human body from a light source behind the body, is reflected by the inner wall of an endoscope lens, and is ultimately guided by an optical fiber to an observation device. An optical lens of the endoscope focuses the light to form a magnified image for the doctor to observe.

It is difficult to maintain the stability of the overall lens sheath with the relevant technology, which leads to situations such as accidental bumping or shaking during the operation of the endoscope, which in turn leads to the optical axis between a rod lens set and an objective lens set being prone to deviation, thereby affecting the clarity of images transmitted via the rod lens set.

### SUMMARY

In view of the above technical problem, it is necessary to provide a protection structure for a lens sheath of an endoscope, capable of reducing a shaking condition and ensuring clarity of image transmission.

A protection structure for a lens sheath of an endoscope includes: an incident light module, an objective lens module, an imaging module, and a lens sheath coaxial module; where the incident light module is configured to provide an incident light source, which strikes onto a surface of an object under observation after passing through the objective lens module, and then after being reflected by the surface of the object under observation, passes through the objective lens module again and propagates backward to the imaging module for imaging processing; where the lens sheath coaxial module includes a base, an internal lens sheath fixedly connected to the base, and an external lens sheath sleeved on an outside of the internal lens sheath and fixedly connected to the base, the objective lens module is fixedly disposed at a front end of the internal lens sheath, and the external lens sheath includes a protective lens fixedly disposed at a front end of the external lens sheath.

In an embodiment of the present disclosure, the internal lens sheath includes a horizontally disposed first lens sheath tube body, and the objective lens module is disposed at a front end of the first lens sheath tube body.

In an embodiment of the present disclosure, the external lens sheath includes a second lens sheath tube body disposed in parallel with the first lens sheath tube body, and a lens sheath connection portion integrally molded at a rear end of the second lens sheath tube body, and where the second lens sheath tube body is fixedly connected to the base via the lens sheath connection portion.

In an embodiment of the present disclosure, the protective lens is provided at a front end of the second lens sheath tube body and is located at a front end of the objective lens module.

In an embodiment of the present disclosure, the protective lens includes a rear cutoff surface and a front cutoff surface, which are perpendicular to an optical axis of the objective lens module.

In an embodiment of the present disclosure, the protective lens further includes a transition surface extending inclinedly backward from an edge of the front cutoff surface, the transition surface extends inclinedly backward to an inner wall of the external lens sheath and then further extends horizontally backward to form a fitting surface, the fitting surface extending backward and cutting off and forming the rear cutoff surface.

In an embodiment of the present disclosure, the front cut-off surface is circular and has a diameter that is not less than an imaging diameter of an objective lens system.

In an embodiment of the present disclosure, a boundary formed by the transition surface and the fitting surface does not protrude beyond a frontmost end of the external lens sheath.

In an embodiment of the present disclosure, a frontmost end of the external lens sheath does not protrude beyond the front cutoff surface.

In an embodiment of the present disclosure, a hermetic space is formed between the internal lens sheath and the external lens sheath.

In an embodiment of the present disclosure, a distance between the front cutoff surface and the rear cutoff surface does not exceed a working distance of the objective lens module, and is not less than 1.5 mm.

In an embodiment of the present disclosure, a material of the protective lens is any one or more of: glass, quartz, plastic film, glass filled with water inside, quartz filled with water inside, plastic film filled with water inside.

In an embodiment of the present disclosure, a distance between the rear cutoff surface and a frontmost end of the objective lens module is 0.1 mm ± 0.05 mm.

The above protection structure for the lens sheath of the endoscope includes: an incident light module, an objective lens module, an imaging module, and a lens sheath coaxial module; where the incident light module is configured to provide an incident light source, which strikes onto a surface of an object under observation after passing through the objective lens module, and then after being reflected by the surface of the object under observation, passes through the objective lens module again and propagates backward to the imaging module for imaging processing; where the lens sheath coaxial module includes a base, an internal lens sheath fixedly connected to the base, and an external lens sheath sleeved on an outside of the internal lens sheath and fixedly connected to the base, the objective lens module is fixedly disposed at a front end of the internal lens sheath, and the external lens sheath includes a protective lens fixedly disposed at a front end of the external lens sheath. The protection structure for the lens sheath of the endoscope presses against the living organism and fixes each module in the endoscope system, the incident light module guides the incident light to irradiate the object surface of the objective lens module, and the objective lens module is used to generate fluorescence by the excitation light on the object surface thereof so as to image, which is capable of improving the stability of the endoscope system, and is conducive to generating a clearer and more accurate image to facilitate the medical personnel to observe the living organism and to improve the accuracy and efficiency of the medical examination.

### BRIEF DESCRIPTION OF THE DRAWINGS

In order to more clearly illustrate the technical solutions in the embodiments of the present application or in the conventional technology, the accompanying drawings to be used in the description of the embodiments or the related technology will be briefly introduced below, and it will be obvious that the accompanying drawings in the following description are only for embodiments of the present application, and that other accompanying drawings can be obtained according to the disclosed accompanying drawings for the person of ordinary skill in the field without creative labors.
FIG. 1 shows a schematic diagram of a cross-section of a protective lens in a protection structure for a lens sheath of an endoscope according to an embodiment;
FIG. 2 shows a schematic diagram of a cross-section of a lens sheath coaxial module in a protection structure for a lens sheath of an endoscope according to an embodiment;
FIG. 3 shows a schematic diagram of a cross-section of an incident light module in a protection structure for a lens sheath of an endoscope according to an embodiment;
FIG. 4 shows a schematic diagram of a cross-section of a fluorescence module in a protection structure for a lens sheath of an endoscope according to an embodiment;
FIG. 5 shows a schematic structural diagram of a self-impacted structure employed for a dichroscope according to an embodiment;
FIG. 6 shows a schematic diagram of a cross-section of an objective lens module in a protection structure for a lens sheath of an endoscope according to an embodiment;
FIG. 7a shows a schematic diagram of a cross-section of a front end of a relay lens module in a protection structure for a lens sheath of an endoscope according to an embodiment;
FIG. 7b shows a schematic diagram of a cross-section of a rear end of a relay lens module in a protection structure for a lens sheath of an endoscope according to an embodiment;
FIG. 8 shows a schematic diagram of a cross-section of a rear-end magnifying lens module in a protection structure for a lens sheath of an endoscope according to an embodiment;
FIG. 9a shows a front view of a protection structure for a lens sheath of an endoscope according to an embodiment;
FIG. 9b shows a right view of a protection structure for a lens sheath of an endoscope according to an embodiment;
FIG. 9c shows an upper view of a protection structure for a lens sheath of an endoscope according to an embodiment; and
FIG. 10 shows a schematic diagram of a cross-section of a protection structure for a lens sheath of an endoscope according to an embodiment.

### DETAILED DESCRIPTION OF THE EMBODIMENTS

An endoscope is a device for examining and treating internal tissues of human body by means of a head probe and an optical lens, whose working principle is based on optical imaging and probe technology, which is used to observe microscopic structures or diseased tissues in the body by introducing a light source and a lens into cavities or tissues of the body. Among other things, the optical imaging principle of the endoscope means that light enters the human body from the light source behind the body, is reflected by an inner wall of an endoscopic lens, and is ultimately guided by an optical fiber to an observation element, and an optical lens of the endoscope focuses the light to form a magnified image for a doctor to observe.

The realization of the optical imaging of the endoscope mainly depends on an optical system provided inside the endoscope. Taking an end where living biological tissue is located as front end, the optical system includes an objective lens, a relay lens set, and an eyepiece lens which are arranged from front end to back end. The objective lens is arranged to collect information of the living biological tissue and generate an image based on the collected information. The relay lens set is arranged to transmit the image. The eyepiece lens is arranged to magnify the image for observation by a clinician. The basis of optical imaging is the information of the living biological tissue collected by the objective lens, so the collection of the information of the living biological tissue is an important and indispensable part.

It is difficult to maintain the stability of an overall lens sheath in the related technology, which leads to actions such as accidental bumping or shaking during operation, making an optical axis between a rod lens set and an objective lens set prone to offset, thereby adversely affecting the clarity of image transmission by the rod lens set. Here, the rod lens is a rod-like lens. Therefore, it is necessary to design an overall imaging system with which influence of shaking on the image can be reduced.

Technical solutions in embodiments of the present disclosure are described clearly and completely hereinafter in conjunction with accompanying drawings used in the embodiments of the present disclosure. It is obvious that described embodiments are only a part of rather than all of embodiments of the present application. Based on the embodiments of the present disclosure, all other embodiments obtained by a person of ordinary skill in the art without making creative labor all fall within the scope of protection of the present application.

In the description of the present disclosure, it is to be understood that the terms "center", "longitudinal", "lateral", "length ", "width", "thickness", "on", "under", "front", "back", "left", "right", "vertical ", "horizontal", "top", "bottom", "inner", "outer", "clockwise", "counterclockwise", "axial", "radial", "circumferential", etc. indicate orientations or positional relationships based on those shown in the accompanying drawings, and are intended only to facilitate the description of the present disclosure and to simplify the description, and are not intended to indicate or imply that the device or element referred to must have a particular orientation, be constructed and operated in a particular orientation, and therefore cannot be be construed as a limitation of the present disclosure.

Furthermore, the terms "first" and "second" are used for descriptive purposes only and are not to be understood as indicating or implying order or relative importance, or implicitly specifying the number of technical features indicated. Thus, a feature defined with "first", "second" may expressly or impliedly include at least one such feature. In the description of the present disclosure, "multiple" means at least two, e.g., two, three, etc., unless otherwise expressly and specifically limited.

In this disclosure, unless otherwise expressly provided and limited, the terms "mount", "communicate", "connect", "fix", and the like are to be broadly construed. For example, it may be a fixed connection, a detachable connection, or integrating as a whole; it may be a mechanical connection, an electrical connection, or a communication connection; it may be a direct connection, an indirect connection through an intermediary medium, a connection within two elements, or an interaction between two elements, unless otherwise expressly limited. For those of ordinary skill in the art, the specific meaning of the above terms in the present disclosure may be understood on a case-by-case basis.

In the present disclosure, unless otherwise expressly provided and limited, the first feature being "on" or "under" the second feature may be a direct contact between the first and second features, or an indirect contact between the first and second features through an intermediate medium. Furthermore, the first feature being "on", "above" and "over" the second feature may be that the first feature is directly above or inclinedly above the second feature, or simply that the first feature is horizontally higher than the second feature. The first feature is "under", "below" and "underneath" the second feature may be that the first feature is directly below or inclinedly below the second feature, or simply that the first feature is horizontally lower than the second feature.

It should be noted that when an element is said to be "fixed to" or "disposed on" another element, it may be directly on the other element or there may be an intermediate element. When an element is said to be "connected" to another element, it may be directly connected to the other element or there may be an intermediate element. As used herein, the terms "vertical", "horizontal", "top", "bottom", "left," "right," and similar expressions are used herein for illustrative purposes only and are not intended to be the exclusive means of implementation.

A protection structure for a lens sheath of an endoscope is provided according to an embodiment. The endoscope system includes an incident light module, an objective lens module, an imaging module, and a lens sheath coaxial module. The incident light module is arranged to provide an incident light source, which strikes onto a surface of an object under observation after passing through the objective lens module, and then after being reflected by the surface of the object under observation, passes through the objective lens module again and propagates backward to the imaging module for imaging processing. The lens sheath coaxial module includes a base, an internal lens sheath fixedly connected to the base, and an external lens sheath sleeved on the outside of the internal lens sheath and fixedly connected to the base. The objective lens module is fixedly disposed at a front end of the internal lens sheath. The external lens sheath includes a protective lens fixedly disposed at a front end of the external lens sheath.

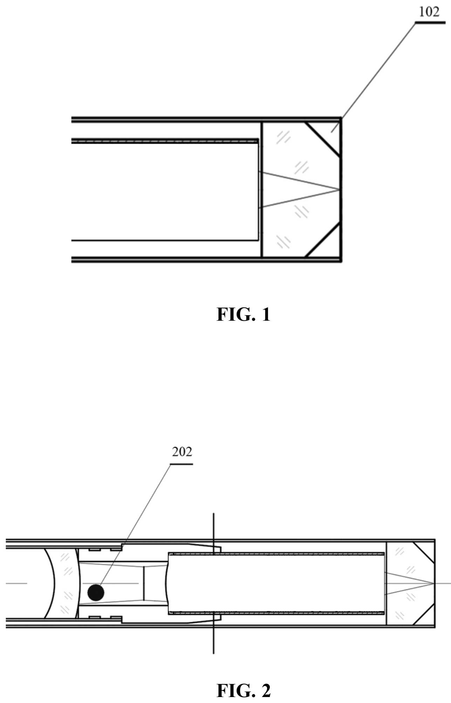

Specifically, as shown in FIG. 1, a layer of protective lens 102 is provided between the front end of the external lens sheath and the object under observation, for preventing the endoscope from shaking during use. A lens sheath terminal in the lens sheath coaxial module extends beyond the protective lens to fit onto a surface of a living organism under observation.

Exemplarily, the imaging module in the protection structure for the lens sheath of the endoscope further includes a relay lens module, a fluorescence module, and a rear-end magnifying lens module. The incident light module is arranged to provide an incident light source and guide the incident light into the fluorescence module to generate an excitation light. The fluorescence module is arranged to reflect the excitation light to the relay lens module and the objective lens module, to irradiate an object surface of the objective lens module and the surface of the object under observation. The objective lens module is arranged to generate fluorescence due to excitation of the excitation light on the object surface of the objective lens module. After being reflected by the surface of the object under observation, the fluorescence propagates to the fluorescence module and the rear-end magnifying lens module via the objective lens module and the relay lens module, i.e., passes through the objective lens module and propagates backward to the imaging module for imaging processing.

Furthermore, the lens sheath coaxial module is arranged to secure respective modules in the endoscope system.

Specifically, as shown in FIG. 2, the lens sheath coaxial module includes a lens sheath 202 and a coaxial clamping groove module (not shown in FIG. 2). Positions of respective lenses are effectively fixed by a coaxial clamping groove system at a front end inside the lens sheath through clamping. At the same time, the rod lens set and the objective lens set are held at a solid distance from each other by the coaxial clamping groove module, without displacement, and are overall on an identical circular axis.

Specifically, on the basis of the internal lens sheath, a layer of protective lens sheath, i.e., the external lens sheath is sleeved on the outside of the internal lens sheath. The protective lens sheath is detachable for ensuring the stability of the overall lens sheath. Since the protective lens sheath is detachable, it is selectable to install or detach the protective lens sheath according to needs of different scenarios when using the endoscope system, improving the flexibility of the endoscope system.

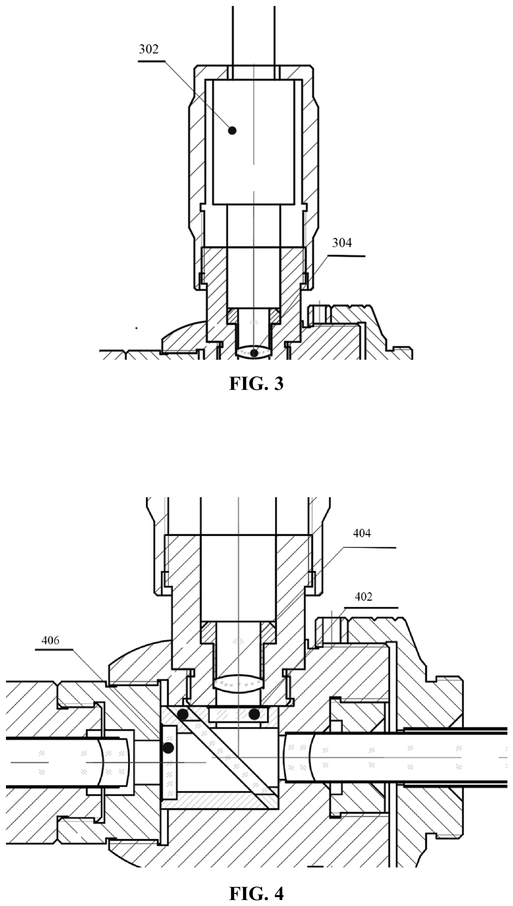

Specifically, as shown in FIG. 3, the incident light module includes an incident optical fiber 302 and an illumination lens 304. Light emitted through the incident optical fiber 302 passes through the illumination lens 304 and enters an excitation filter in the fluorescence module. The incident light is emitted through the incident optical fiber, gets collected by the illumination lens, and then enters the fluorescence module. From an exit end face of the incident optical fiber, the incident light is imaged to a system diaphragm of an entire microendoscope through a combined optical system of the illumination lens and a part of the relay lens module; that is to say, there exists an object-image relationship between the exit end face of the incident optical fiber and the system diaphragm of the entire microendoscope, so as to facilitate the realization of Koehler illumination. The Koehler illumination enables uniform and sufficiently bright illumination onto the living biological tissue to be examined, and does not cause dazzling glare.

Specifically, as shown in FIG. 4, the fluorescence module includes an excitation filter 402, a dichroscope 404, and a fluorescence filter 406, where the fluorescence filter may be replaced by an emission filter. The incident light is emitted through the incident optical fiber in the incident light module and enters the fluorescence module of the endoscope system, an excitation light is obtained through the excitation filter 402 in the fluorescence module, the dichroscope 404 reflects the excitation light into the relay lens module, and then the excitation light enters the objective lens module from the relay lens module and uniformly illuminates a position of the object surface. The excitation light excites a fluorescence of the living biological tissue, and fluorescence information of the living biological tissue is collected by the objective lens and imaged onto an image plane of the objective lens; the fluorescence is further transmitted over a long distance by the relay lens module, filtered by the fluorescence filter 406 after passing through the dichroscope 404, and transmitted to the rear-end magnifying lens module.

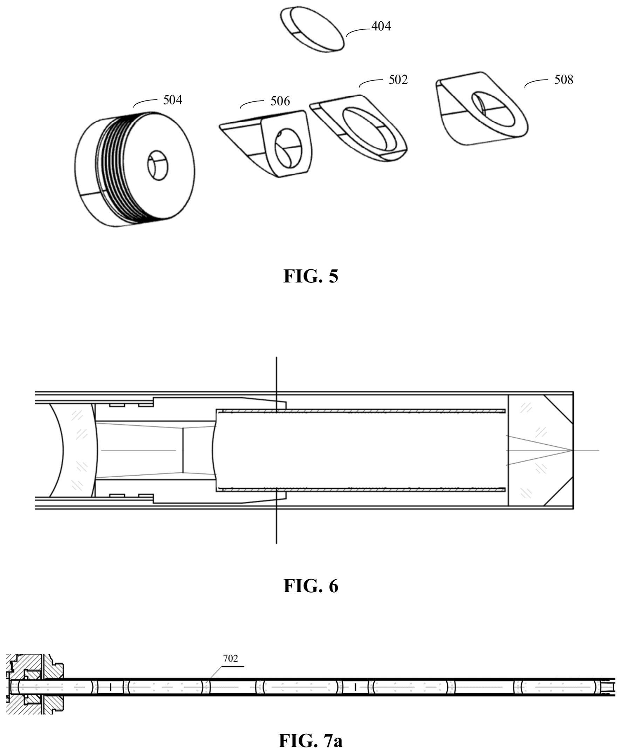

Further, as shown in FIG. 5, a self-impacted structure includes the dichroscope 404, a metal washer 502, a fixing connector 504, an upper-left half-triangular fixing member 506, and a lower-right half-triangular fixing member 508. Two half-triangular tapered fixing members, i.e., the upper-left half-triangular fixing member 506 and the lower-right half-triangular fixing member 508 that hold the dichroscope 404 therebetween, as well as the metal washer 502 combine to form a fixing structure assembly for the dichroscope 404 located therein, where the fixing structure assembly is accommodated in an intermediate accommodating cavity. A left side of the upper-left half-triangular fixing member 506 abuts against a left side of an inner wall of the accommodating cavity, and a right side of the inner wall of the accommodating cavity impacts, through the fixing connector 504, an entire assembly of dichroscope 404 within the accommodating cavity. The fixing connector 504 may be fixedly connected to the accommodating cavity by means of, for example, screwing, riveting or the like. Optionally, the dichroscope 404 is tilted at an angle of 45 degrees. Since the dichroscope in the fluorescence module is installed in the endoscope system by adopting the self-impacted structure, it is possible to ensure that the dichroscope may not suffer from accidental failures such as positional deviation during use and thus affect the image quality.

Specifically, the fluorescence module includes the excitation filter, the dichroscope and the fluorescence filter. A light path of the endoscope system is that: the incident light enters the fluorescence module through the incident light module, the excitation light is generated by the excitation filter from the incident light, the excitation light is reflected by the dichroscope to the relay lens module and the objective lens module and irradiates the object surface of the objective lens module, fluorescence is generated after the object surface of the objective lens module is excited by the excitation light, the fluorescence propagates over the objective lens module and the relay lens module, passes through the dichroscope and gets filtered by the fluorescence filter, and then enters the rear-end magnifying lens module, so that incident light and emergent light propagate in an identical tubular cavity, and at the same time, it realizes the filtration of a specific fluorescence wave band.

Specifically, in order to ensure that the image of the objective lens module can be transmitted over a long distance, the objective lens module adopts an image-side telecentric microscope objective system with a certain magnification. From the technical principle consideration, the higher the telecentricity of the image-side telecentric microscope objective system, the more helpful for imaging.

Specifically, the objective lens module includes a small objective lens, and the fluorescence is imaged once by the small objective lens, and then imaged by the relay lens module in accordance with a target number of times, and enters the rear-end magnifying lens module. Further, as shown in FIG. 6, the objective lens module includes a small objective lens, and the excitation light emitted by the relay lens module passes through the objective lens module and uniformly illuminates the object surface, and at this time, light rays are not necessarily parallel; the fluorescence of the living biological tissue is excited, and the fluorescence information of the living biological tissue is collected by the objective lens module and imaged onto the image plane of the objective lens module, and then transmitted over a long distance by the relay lens module to the rear-end magnifying lens module.

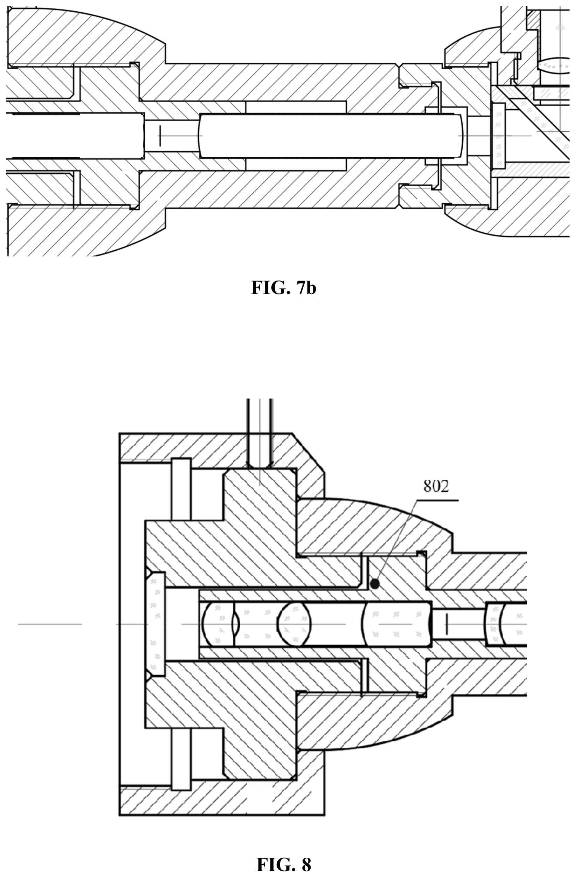

Specifically, the relay lens module includes a relay lens, the relay lens includes a rod lens set, each rod lens set includes two groups of rod lenses, and the two groups of rod lenses form a dual-telecentric imaging system with a target magnification. As shown in FIG. 7a and FIG. 7b, FIG. 7a shows a front end of the relay lens module, and FIG. 7b shows a rear end of the relay lens module, where 702 donates the rod lens, i.e., glued triplet lens, and the two groups of rod lenses form a dual-telecentric-1x imaging system, i.e., the magnification is 1. Multiple dual-telecentric imaging systems combine to form the relay lens, i.e., the relay lens includes even number of groups of rod lenses. An optical path between the rod lenses of the dual telecentric imaging system is quasi-parallel, i.e., not necessarily an absolutely parallel optical path. Multiple groups of rod lenses are combined to form the relay lens, two groups of rod lenses form the dual telecentric-1x imaging system, and the overall magnification of the relay lens is 1x or -1x. Because the rod lenses are relatively small and lightweight, they can be easily held and manipulated, and the observer can use them more flexibly; the rod lenses can also provide a larger field of view, and observation can be performed in a narrow or difficult-to-reach space. The use of multiple groups of rod lenses to form the relay lens can realize the long-distance transmission of images, so that the object can be observed at a long distance, reducing the risk of close observation and avoiding the interference of the living organism to be examined.

Specifically, one particular dual-telecentric imaging system is selected from the relay lens, and the fluorescence module is placed in the parallel optical path of the selected dual-telecentric imaging system. Since the effect of the placement of the fluorescence module on the original relay lens imaging optical path is very small and almost negligible, placing the fluorescence module between the rod lenses that form a target dual-telecentric imaging system, while realizing the functions of generating the excitation light and filtering the fluorescence, etc., does not affect the original optical path, thereby reducing adverse effect on the image quality.

Specifically, as shown in FIG. 8, the rear-end magnifying lens module includes a combination 802 of a magnifying lens and a rod lens, and an object plane of the magnifying lens coincides with the image plane of the relay lens to realize magnification of a generated image and obtain a clearer image, thereby facilitating medical examination.

Specifically, a camera device includes a Complementary Metal Oxide Semiconductor, CMOS, camera. An object-side size of the magnifying lens corresponds to an image-side size of the camera device. Another function of the magnifying lens is to modulate an angle of main light rays emergent from the relay lens, so as to match an angle of main light rays of the CMOS, and to enhance fluorescence collection energy. By setting up the camera device to perform fluorescence imaging, it is facilitated to obtain a clearer and more accurate examination image; the camera device includes a CMOS camera, and imaging using the CMOS camera has many advantages, not only in terms of production cost, but also CMOS has a shorter manufacturing cycle and better scalability. At the same time, CMOS can achieve better low-light and high-light control, with higher superiority and higher stability in high-resolution small devices, so it is suitable for endoscope systems. In addition, the magnifying lens has a certain magnification so that the object-side size corresponds to the CMOS image-side size, enabling maximum utilization of the pixels.

Specifically, the endoscope system further includes an image processing module for processing an image formed on the camera device and outputting processed image data. Further, fluorescence entering the magnifying lens is directly imaged on the CMOS camera, and through the image processing module, an enlarged and clear image picture is output to improve the quality of the image picture, thereby enhancing the accuracy of the medical examination.

In the embodiment, the protection structure for the lens sheath of the endoscope includes the incident light module, the objective lens module, the imaging module, and the lens sheath coaxial module. The incident light module is used to provide an incident light source, which strikes onto a surface of an object under observation after passing through the objective lens module, and then after being reflected by the surface of the object under observation, passes through the objective lens module again and propagates backward to the imaging module for imaging processing, realizing the medical examination of the living organism to be examined, avoiding a shaking situation, thereby efficiently and accurately obtaining a clear examination image. The lens sheath coaxial module includes a base, an internal lens sheath fixedly connected to the base, and an external lens sheath sleeved on the outside of the internal lens sheath and fixedly connected to the base. The objective lens module is fixedly located at a front end of the internal lens sheath. The external lens sheath includes a protective lens fixedly located at a front end of the external lens sheath. With the lens sheath coaxial module, the lenses in the endoscope system are effectively clamped and fixed, ensuring the stability of the endoscope system and avoiding problems such as falling off and disintegration in the process of use.

In an embodiment of the present disclosure, the internal lens sheath includes a horizontally disposed first lens sheath tube body, and the objective lens module is disposed at a front end of the first lens sheath tube body.

In the embodiment, the first lens sheath tube body is used to secure the objective lens module.

In an embodiment of the present disclosure, the external lens sheath includes a second lens sheath tube body disposed in parallel with the first lens sheath tube body, and a lens sheath connection portion integrally molded at a rear end of the second lens sheath tube body, the second lens sheath tube body being fixedly connected to the base via the lens sheath connection portion.

Specifically, a fixing connection approach includes threaded connection, snap connection, plug-in connection, welded connection, adhesive connection, and the like.

In the embodiment, the second lens sheath tube body is fixedly connected to the base via the lens sheath connection portion, which facilitates fixing the protective lens disposed at the front end of the external lens sheath.

In an embodiment of the present disclosure, the protective lens is provided at the front end of the second lens sheath tube body and is located at the front end of the objective lens module.

In the embodiment, the protective lens is designed at the front end of the second lens sheath tube body and at the front end of the objective lens module, and the design point of the width of the protective lens is to ensure that the incident light will converge on the surface of the object under observation after the incident light is emitted from the objective lens module, and that the negative effects caused by shaking can be effectively prevented by pressing the protective lens against the surface of the living organism under observation. On the other hand, a terminal of the lens sheath extends beyond the protective lens, and when attached to the surface of the living organism, a periphery of the terminal of the lens sheath abuts against a periphery of the observation area, which further prevents the negative effects of shaking.

In an embodiment of the present disclosure, the protective lens includes a rear cutoff surface and a front cutoff surface, which are perpendicular to the optical axis of the objective lens module.

In the embodiment, the front cutoff surface and the rear cutoff surface together define the thickness of the protective lens, and the thickness of the protective lens has a corresponding effect on the imaging quality of the endoscope.

In an embodiment of the present disclosure, the protective lens further includes a transition surface extending inclinedly backward from an edge of the front cutoff surface, the transition surface extends inclinedly backward to the inner wall of the external lens sheath and then further extends horizontally backward to form a fitting surface, the fitting surface extending backward and cutting off and forming the rear cutoff surface.

In the embodiment, the protective lens extends to the inner wall of the external lens sheath by the transition surface and forms the fitting surface, and the fitting surface extends backwardly to form the rear cutoff surface, thereby ensuring that the protective lens is more firmly tightly-fastened to the external lens sheath.

In an embodiment of the present disclosure, the front cutoff surface is circular and has a diameter that is not less than an imaging diameter of an objective lens system.

In the embodiment, defining the shape and diameter of the front cutoff surface is a prerequisite for ensuring that the endoscope is able to image.

In an embodiment of the present disclosure, a boundary formed by the transition surface and the fitting surface does not protrude beyond the frontmost end of the external lens sheath.

In the embodiment, since the protective lens is provided at the front end of the external lens sheath, and the transition surface of the protective lens extends inclinedly backward to the inner wall of the external lens sheath and further extends horizontally backward to form the fitting surface, the boundary formed by the transition surface and the fitting surface does not protrude beyond the frontmost end of the external lens sheath.

In an embodiment of the present disclosure, the frontmost end of the external lens sheath does not protrude beyond the front cutoff surface.

In the embodiment, since at the part of peripheral fitting surface of the protective lens, there may have non-imaging light entering the protective lens from a lateral side, thereby affecting the endoscopic imaging, the fitting surface of the lateral side of the protective lens must be fully contracted, and the protective lens and the inner wall of the external lens sheath must be sealingly bonded by means of glue or the like to ensure the stability of the protective lens. If the frontmost end of the external lens sheath protrudes beyond the front cutoff surface, endoscopic imaging will be affected, and at the same time pose a risk to the surgery. When the endoscope is used for imaging operations, the front cutoff surface of the protective lens is adhered to the surface of the living tissue and needs to continuously slid on the surface of the living tissue, if the frontmost end of the external lens sheath protrudes beyond the front cutoff surface, the periphery of the lens sheath will cause additional pressure on the surface of the living tissue, which will cause damage to the living tissue, which will not only cause a series of risks, but will also fail to realize the continuous sliding of the endoscope. In the present embodiment, the biological beating that comes with the living body can be reduced by pressing the front face against the surface of the living body, and an exceeding portion of the external lens sheath can also play a role in assisting in fixing the observation area during the imaging process, which likewise further ensures the stability of the imaging, and plays a role in preventing the shaking.

In an embodiment of the present disclosure, a hermetic space is formed between the internal lens sheath and the external lens sheath.

In the embodiment, the internal lens sheath and the external lens sheath form the hermetic space therebetween, ensuring the safety of the internal lens sheath. Since the sterilization requirements for endoscopes in the clinic are very demanding, once the internal lens sheath and the external lens sheath are not hermetic, the internal lens sheath and the lens will be contaminated. In addition, the external lens sheath can be replaced, and its disposable replacement property can reduce the cost of the operation and ensure safety.

In an embodiment of the present disclosure, a distance between the front cutoff surface and the rear cutoff surface does not exceed a working distance of the objective lens module, and is not less than 1.5 mm.

Specifically, the distance between the front cutoff surface and the rear cutoff surface, i.e., the thickness of the protective lens, depends on the working distance of the small objective lens in the objective lens module, with a thickest thickness not exceeding the working distance of the small objective lens, and a thinnest thickness not lower than 1.5 mm, i.e., 1.5 mm ≤ thickness of the protective lens ≤ working distance of the small objective lens in the objective lens module.

In an embodiment, in order to be able to add an anti-shaking lens sheath structure to the objective lens module, the objective lens module will first be set up as an objective lens system with negative spherical aberration, and the spherical aberration will be compensated for by a dielectric lens as the anti-shaking structure. And determining the distance between the front cutoff surface and the rear cutoff surface based on the working distance of the small objective lens can avoid the risk of rupture faced by the protective lens due to being too thin while realizing accurate imaging. In embodiments of the present disclosure, the material of the protective lens is any one or more of: glass, quartz, plastic film, glass filled with water inside, quartz filled with water inside, and plastic film filled with water inside.

Specifically, glass is selected as the material of the protective lens, and other light-transmitting homogeneous media materials may be used instead, i.e., quartz, plastic film, glass filled with water inside, quartz filled with water inside, plastic film filled with water inside.

In the embodiment, by selecting any one or more of glass, quartz, plastic film, glass filled with water inside, quartz filled with water inside, plastic film filled with water inside as the material of the protective lens, the material of the protective lens is expanded, which can reduce the influence of the protective lens on the fluorescent image, realize clearer imaging, and at the same time reduce the cost of the protective lens.

In an embodiment of the present disclosure, a distance between the rear cutoff surface and the frontmost end of the objective lens module is 0.1 mm ± 0.05 mm.

In the embodiment, in consideration of the process expansion, the rear cutoff surface and the frontmost end of the objective lens module cannot be completely affixed to each other, and there will maintain a certain gap, so the distance between the rear cutoff surface and the frontmost end of the objective lens module is further described.

In the embodiments of the present disclosure, a protection structure for a lens sheath of an endoscope is provided. The endoscope system includes an incident light module, an objective lens module, a relay lens module, a fluorescence module, a rear-end magnifying lens module, and a lens sheath coaxial module. The incident light module guides the incident light into the fluorescence module to obtain an excitation light; the incident light module includes an incident optical fiber and an illumination lens, and the incident light enters the fluorescence module through the incident optical fiber and the illumination lens. The fluorescence module is used to reflect the excitation light to the relay lens module and objective lens module to irradiate the object surface of the objective lens module. The fluorescence module includes an excitation filter, a dichroscope and a fluorescence filter, the dichroscope adopts self-impacted structure. The objective lens module is used to produce fluorescence by the excitation light on its object surface, the objective lens module adopts the image-side telecentric microscope objective system, the objective lens module includes a small objective lens, the fluorescence is imaged by the small objective lens once, and then imaged by the relay lens module in accordance with a target number of times. The relay lens module includes a relay lens, the relay lens includes a rod lens set, where each rod lens set includes two groups of rod lenses, and the two groups of rod lenses form a dual-telecentric imaging system with a target magnification. A target dual-telecentric imaging system is selected, and the fluorescence module is located between the rod lenses forming the target dual-telecentric imaging system. The fluorescence module is also used for filtering fluorescence, and the filtered fluorescence enters a rear-end magnifying lens module. The rear-end magnifying lens module includes a magnifying lens, the object plane of the magnifying lens coincides with the image plane of the relay lens. The rear-end magnifying lens module also includes a camera device, and the fluorescence is imaged on the camera device. The lens sheath coaxial module includes a base, an internal lens sheath that is fixedly connected to the base, and an external lens sheath sleeved on the outside of the internal lens sheath and fixedly connected to the base. The objective lens module is fixedly disposed at a front end of the internal lens sheath, and the external lens sheath includes a protective lens fixedly located at a front end of the external lens sheath. The internal lens sheath includes a horizontally disposed first lens sheath tube body, and the objective lens module is set at the front end of the first lens sheath tube body; the external lens sheath includes a second lens sheath tube body disposed in parallel with the first lens sheath tube body, and a lens sheath connection portion integrally molded at a rear end of the second lens sheath tube body, the second lens sheath tube body being fixedly connected with the base through the lens sheath connection portion. The protective lens is provided at the front end of the second lens sheath tube body and is located at the front end of the objective lens module. The protective lens includes a rear cutoff surface and a front cutoff surface, which are perpendicular to the optical axis of the objective lens module. The protective lens also includes a transition surface extending inclinedly backward from an edge of the front cutoff surface, the transition surface extends inclinedly backward to the inner wall of the external lens sheath and then further extends horizontally backward to form a fitting surface, the fitting surface extending backward and cutting off and forming the rear cutoff surface. The front cutoff surface is circular, and its diameter is not smaller than an imaging diameter of an objective lens system. A boundary formed by the transition surface and the fitting surface does not protrude beyond the frontmost end of the external lens sheath; the frontmost end of the external lens sheath does not protrude beyond the front cutoff surface. A hermetic space is formed between the internal lens sheath and the external lens sheath, and the distance between the front cutoff surface and the rear cutoff surface is not more than the working distance of the objective lens module, as well as being not less than 1.5 mm. The material of the protective lens is any one or more of glass, quartz, plastic film, glass filled with water inside, quartz filled with water inside, and plastic film filled with water inside, and the distance between the rear cutoff surface and the frontmost end of the objective lens module is 0.1 mm ± 0.05 mm. The lens sheath coaxial module also includes a detachable protective lens sheath, and the protective lens sheath is used to ensure the stability of the overall lens sheath. Further, the endoscope system further includes an image processing module for processing the image formed on the camera device and outputting the processed image data.

In some embodiments, the incident light enters the endoscope system through the incident optical fiber, and enters the fluorescence module through the illumination lens. The incident light exits the illumination lens, is filtered by an excitation filter, then reflected by a dichroscope to enter a relay lens, and then enters a small objective lens to uniformly illuminate the position of the object surface. The object surface is excited by the excitation light to produce fluorescence, the fluorescence information emitted by living biological tissues is imaged once by the small objective lens, and then imaged several times by the relay lens into the magnifying lens. The dichroscope and the fluorescence filter are located between the relay lenses. The fluorescence is filtered by the fluorescence filter after passing through the dichroscope, and fluorescence into the magnifying lens is directly imaged on the CMOS camera, and the enlarged and clear image is output through the image processing system.

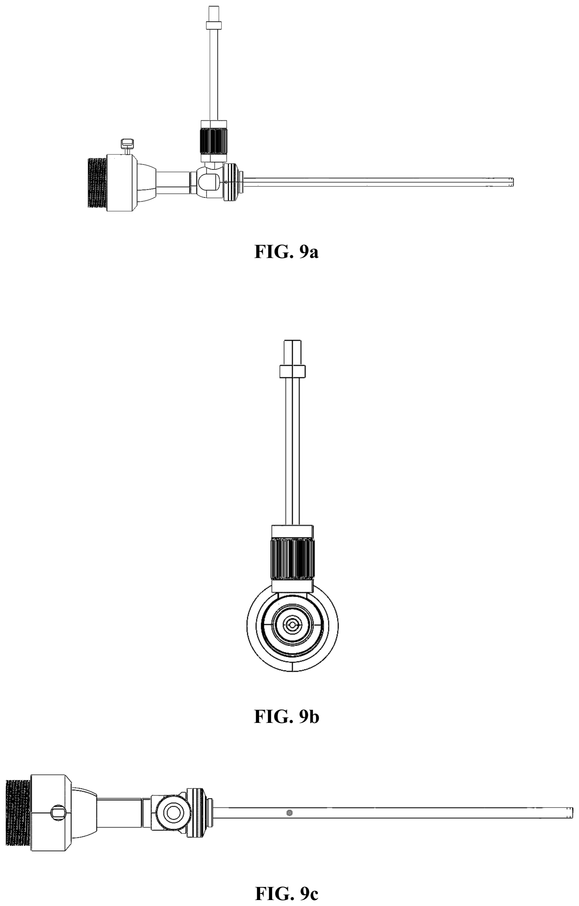

In some embodiments, three-view drawings of a protection structure for a lens sheath of an endoscope is provided as shown in FIGS. 9a, 9b, and 9c, where FIG. 9a shows a front view of the protection structure for the lens sheath of the endoscope, FIG. 9b shows a right view of the protection structure for the lens sheath of the endoscope, and FIG. 9c shows an upper view of the protection structure for the lens sheath of the endoscope.

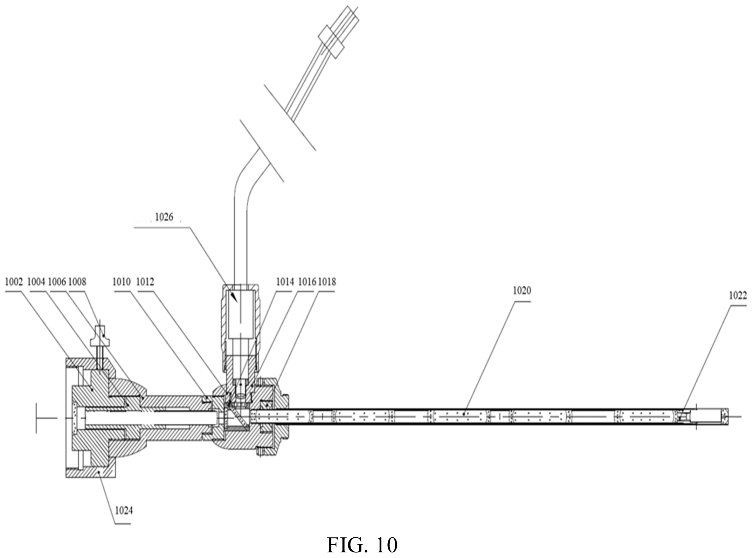

Further, FIG. 10 shows a sectional structure of a protection structure for a lens sheath of an endoscope, including a camera holder component 1002, a 4x magnifying lens and rod lens set 1004, a connecting tube 1006, a pin 1008, a pressure ring 1010, a lens body 1012, a fluorescence module 1014, a cold light source coaxial illuminator 1016, a connecting ring 1018, a rod lens component and a 3X0.45 small objective lens 1020, lens sheath 1022, camera pressure cap 1024, and illumination optical fiber 1026, where the illumination optical fiber is the incident optical fiber.

The above-described embodiments and the various technical features in the above-described embodiments may be combined arbitrarily, and all possible combinations of the various technical features in the above-described embodiments have not been described for the sake of simplicity of description; however, as long as the combinations of these technical features are not contradictory, they should be considered to be within the scope of the present specification as recorded herein.

The above-described embodiments express only several embodiments of the present application, which are described in a more specific and detailed manner, but are not to be construed as a limitation of the scope of the patent application. It should be pointed out that, for a person of ordinary skill in the art, several deformations and improvements can be made without departing from the conception of the present application, all of which fall within the scope of protection of the present application. Therefore, the scope of protection of the patent application shall be subject to the attached claims.

## Claims

1. A protection structure for a lens sheath of an endoscope, **characterized by** comprising: an incident light module, an objective lens module, an imaging module, and a lens sheath coaxial module; wherein the incident light module is configured to provide an incident light source, which strikes onto a surface of an object under observation after passing through the objective lens module, and then after being reflected by the surface of the object under observation, passes through the objective lens module again and propagates backward to the imaging module for imaging processing; wherein the lens sheath coaxial module comprises a base, an internal lens sheath fixedly connected to the base, and an external lens sheath sleeved on an outside of the internal lens sheath and fixedly connected to the base, the objective lens module is fixedly disposed at a front end of the internal lens sheath, and the external lens sheath comprises a protective lens fixedly disposed at a front end of the external lens sheath.

2. The protection structure for the lens sheath of the endoscope according to claim 1, wherein the internal lens sheath comprises a horizontally disposed first lens sheath tube body, and the objective lens module is disposed at a front end of the first lens sheath tube body.

3. The protection structure for the lens sheath of the endoscope according to claim 2, wherein the external lens sheath comprises a second lens sheath tube body disposed in parallel with the first lens sheath tube body, and a lens sheath connection portion integrally molded at a rear end of the second lens sheath tube body, and wherein the second lens sheath tube body is fixedly connected to the base via the lens sheath connection portion.

4. The protection structure for the lens sheath of the endoscope according to claim 3, wherein the protective lens is provided at a front end of the second lens sheath tube body and located at a front end of the objective lens module.

5. The protection structure for the lens sheath of the endoscope according to claim 4, wherein the protective lens comprises a rear cutoff surface and a front cutoff surface, which are perpendicular to an optical axis of the objective lens module.

6. The protection structure for the lens sheath of the endoscope according to claim 5, wherein the protective lens further comprises a transition surface extending inclinedly backward from an edge of the front cutoff surface, the transition surface extends inclinedly backward to an inner wall of the external lens sheath and then further extends horizontally backward to form a fitting surface, the fitting surface extending backward and cutting off and forming the rear cutoff surface.

7. The protection structure for the lens sheath of the endoscope according to claim 6, wherein the front cutoff surface is circular and has a diameter not less than an imaging diameter of an objective lens system.

8. The protection structure for the lens sheath of the endoscope according to claim 6, wherein a boundary formed by the transition surface and the fitting surface does not protrude beyond a frontmost end of the external lens sheath.

9. The protection structure for the lens sheath of the endoscope according to claim 6, wherein a frontmost end of the external lens sheath does not protrude beyond the front cutoff surface.

10. The protection structure for the lens sheath of the endoscope according to claim 6, wherein a hermetic space is formed between the internal lens sheath and the external lens sheath.

11. The protection structure for the lens sheath of the endoscope according to claim 6, wherein a distance between the front cutoff surface and the rear cutoff surface does not exceed a working distance of the objective lens module, and is not less than 1.5 mm.

12. The protection structure for the lens sheath of the endoscope according to claim 6, wherein a material of the protective lens is any one or more of: glass, quartz, plastic film, glass filled with water inside, quartz filled with water inside, plastic film filled with water inside.

13. The protection structure for the lens sheath of the endoscope according to claim 6, wherein a distance between the rear cutoff surface and a frontmost end of the objective lens module is 0.1 mm ± 0.05 mm.
